# EUROPEAN PATENT SPECIFICATION

(11) **EP 3 251 518 B1**
(45) Date of publication and mention of the grant of the patent: **30.07.2025**
(21) Application number: 16743278.0
(22) Date of filing: 25.01.2016
(51) Int. Cl.: A23D 9/007, A23D 7/00, A23D 9/00, A23L 27/60, A61K 31/23, A61K 31/231, A61K 31/232, A61P 3/02, A61P 43/00, C11C 3/08

(54) **OIL OR FAT**
ÖL ODER FETT
HUILE OU GRAISSE

(30) Priority: 26.01.2015 JP 2015012213
(43) Date of publication of application: 06.12.2017
(73) Proprietor: The Nisshin OilliO Group, Ltd., Tokyo 104-8285 (JP)
(72) Inventor: TSUKIYAMA, Muneo, Kanagawa 239-0832 (JP); TAKEGUCHI, Seiya, Kanagawa 239-0832 (JP); HATANO, Yoshiyuki, Kanagawa 239-0832 (JP); UEHARA, Hidetaka, Kanagawa 239-0832 (JP)
(74) Representative: Dennemeyer & Associates S.A.
(86) International application number: PCT/JP2016/051971
(87) International publication number: WO 2016/121675

(56) References cited:
- WO-A1-2010/052847
- WO-A1-2013/147138
- WO-A1-90/04010
- WO-A2-2008/002643
- JP-A- 2001 172 122
- JP-A- H02 502 010
- JP-A- S61 173 743
- US-A1- 2005 196 512
- US-A1- 2005 196 512
- US-A1- 2011 150 944
- D. W. MATHIESON ET AL: "THE SYNTHESIS OF d-1: 2-BISDECANOYLGLYCEROL AND d-1: 2-BISDODECANOYLGLYCEROL*", JOURNAL OF PHARMACY AND PHARMACOLOGY, vol. 9, no. 1, 1 September 1957 (1957-09-01), LONDON; GB, pages 251 - 254, XP055590902, ISSN: 0022-3573, DOI: 10.1111/j.2042-7158.1957.tb12278.x
- STRAARUP E.M. ET AL.: "Structured Lipids Improve Fat Absorption in Normal and Malabsorbing Rats", J. NUTR., vol. 130, no. 11, 2000, pages 2802 - 2808, XP055468526

## Description

### TECHNICAL FIELD

The present invention relates to fats and oils which include constituent fatty acids containing a high content of n-decanoic acid and have excellent cold resistance.

### BACKGROUND ART

The elderly and patients who are ill or after illness are difficult to sufficiently take normal food, which leads to undernutrition in some cases. For addressing such a problem, various foods which allow sufficient nutrients to be ingested in small ingested amounts have been developed. Especially, when the main focus relies on the supply of energy (calories), food with a higher lipid content increases energy intake efficiency. In addition, foods which deal with a ketogenic diet (for example, JP-T-2010-506587), a diabetic diet, and a low carbohydrate diet are preferably low in sugar.

As the lipid used in the above-described foods, medium chain fatty acid triglycerides (MCTs) are known. Medium chain fatty acids contained in MCTs are immediately absorbed in the gastrointestinal tract, and then extremely quickly degraded in the liver into energy. For this reason, MCT is quickly utilized as an energy source after meals. However, when large amounts of medium chain fatty acid triglycerides are ingested at one time, there may be caused discomfort such as irritation of the pharynx or the upper stomach, or heavy feelings in the stomach.

It is said that, among medium chain fatty acid triglycerides (MCTs), MCTs having constituent fatty acids which contain a large amount of n-decanoic acid having 10 carbons scarcely cause the above-described discomfort such as irritation of the pharynx or the upper stomach, or heavy feelings in the stomach (for example, Re-publication of PCT International Publication No. 2010/052847). However, even though the MCTs having a high content of n-decanoic acid as constituent fatty acids are liquid at room temperature, oil and fat crystals are likely to be deposited at a temperature zone around 0 to 5°C. Especially, emulsified foods required to be kept refrigerated, always or only after opening, have the drawback that the ease of use is poor.

### CITATION LIST

### PATENT LITERATURE

Patent Literature 1: JP-T-2010-506587
Patent Literature 2: Re-publication of PCT International Publication No. 2010/052847
Patent Literature 3: US 2005/196512
Patent Literature 4: US 2011/150944
Patent Literature 5: WO 2008/002643
Patent Literature 6: JP S61-173743
Patent Literature 7: Journal of Pharmacy and Pharmacology vol. 9, no. 1 (1957), p.251-254
Patent Literature 8: WO 90/04010

### SUMMARY OF THE INVENTION

### PROBLEMS TO BE SOLVED BY THE INVENTION

An object of the present invention is to provide oils and fats which include constituent fatty acids containing a large amount of n-decanoic acid, scarcely cause discomfort when ingested, and have excellent cold resistance.

### SOLUTION TO THE PROBLEMS

The problem is solved by the compositions according to the claims, which also define the scope of the present invention.

### EFFECTS OF THE INVENTION

According to the present invention, there can be provided oils and fats which include constituent fatty acids containing a large amount of n-decanoic acid, scarcely cause discomfort when ingested, and have excellent cold resistance.

### DESCRIPTION OF THE EMBODIMENTS

The content of medium chain fatty acids contained in constituent fatty acids of the oils and fats according to the present invention is 65 to 95% by mass. As described herein, medium chain fatty acids have a fatty acid having 6 to 10 carbons, and are preferably a linear saturated fatty acid. Specific examples thereof may include n-hexanoic acid (caproic acid), n-octanoic acid (caprylic acid), and n-decanoic acid (capric acid). The content of the medium chain fatty acids contained in the constituent fatty acids of the oils and fats according to the present invention is preferably 70 to 92% by mass, and more preferably 75 to 87% by mass. When the content of the medium chain fatty acids contained in the constituent fatty acids of the oils and fats according to the present invention is within the above-described range, the physiological effect of medium chain fatty acids can be expected.

60 to 75% by mass of medium chain fatty acids of the oils and fats according to the present invention is n-decanoic acid. When the content of n-decanoic acid in the medium chain fatty acids contained in the constituent fatty acids of the oils and fats according to the present invention is within the above-described range, oils and fats having favorable cold resistance can be obtained even when the content of n-decanoic acid is high.

The content of unsaturated fatty acids contained in the constituent fatty acids of the oils and fats according to the present invention is preferably 3 to 35% by mass, more preferably 5 to 30% by mass, and further preferably 6 to 25% by mass. When the content of unsaturated fatty acids contained in the constituent fatty acids of the oils and fats according to the present invention is within the above-described range, oils and fats having favorable cold resistance can be obtained even when the content of n-decanoic acid is high.

An example of the method for identifying and quantitating the composition of the constituent fatty acids and triglycerides of the oils and fats may include analysis by gas chromatography.

The oils and fats according to the present invention include medium chain fatty acid triglycerides (hereinafter, also referred to as MCTs). Medium chain fatty acid triglycerides are a triglyceride in which all of three fatty acids binding to glycerol are a medium chain fatty acid. MCTs contained in the oils and fats according to the present invention can be manufactured by a known method. For example, MCTs can be manufactured by heating fatty acids having 6 to 10 carbons and glycerol to 120 to 180°C, for dehydration and condensation. This condensation reaction is preferably performed under reduced pressure. A catalyst can be used in the above-described condensation reaction. However, the above-described condensation reaction is preferably performed without a catalyst.

The oils and fats according to the present invention also include long chain fatty acid triglycerides (hereinafter, also referred to as LCTs). Long chain fatty acid triglycerides are a triglyceride in which all of three fatty acids binding to glycerol are a long chain fatty acid. The long chain fatty acids according to the present invention are preferably a fatty acid having 12 or more carbons, and a linear fatty acid. More specific examples thereof may include lauric acid, myristic acid, palmitic acid, stearic acid, arachic acid, behenic acid, palmitoleic acid, oleic acid, linoleic acid, and linolenic acid.

70% by mass or more of constituent fatty acids of long chain fatty acid triglycerides contained in the oils and fats according to the present invention is unsaturated fatty acids. Examples of such oils and fats may include soy bean oil, rapeseed oil, corn oil, sesame oil, perilla oil, linseed oil, groundnut oil, safflower oil, sunflower oil, cottonseed oil, grape-seed oil, macadamia nut oil, hazelnut oil, pumpkin seed oil, walnut oil, camellia oil, tea seed oil, perilla oil, borage oil, olive oil, rice oil, rice bran oil, wheat germ oil, and mixed oils and fats, fractionated oils and fats, and transesterified oils and fats thereof.

The oils and fats according to the present invention is also a mixture of MCTs and LCTs. An example thereof may include a mixed oil which contains 65 to 95% by mass (preferably 65 to 85% by mass, and more preferably 65 to 75% by mass) of medium chain fatty acid triglycerides (MCTs), and 5 to 35% by mass (preferably 15 to 35% by mass, and more preferably 25 to 35% by mass) of long chain fatty acid triglycerides (LCTs). The content of n-decanoic acid contained in constituent fatty acids of medium chain fatty acid triglycerides may be 50% by mass or more.

The oils and fats according to the present invention also include medium and long chain fatty acid triglycerides (hereinafter, also referred to as MLCTs). Medium and long chain fatty acid triglycerides are a triglyceride in which three fatty acids binding to the glycerol include at least one medium chain fatty acid and at least one long chain fatty acid. MLCTs may be prepared by esterification, in a similar manner to MCTs. MLCTs may also be prepared by transesterifying a mixture of MCTs and LCTs. The method of transesterification is not particularly limited. A usually practiced method such as chemical transesterification with sodium methoxide as a catalyst, and enzymatic transesterification with a lipase preparation as a catalyst can be applied.

The oils and fats according to the present invention may include MCTs and MLCTs. More specifically, the oils and fats according to the present invention include 30 to 93% by mass of MCTs and 3 to 70% by mass of MLCTs, more preferably 45 to 84% by mass of MCTs and 6 to 55% by mass of MLCTs, and further preferably 60 to 78% by mass of MCTs and 8 to 40% by mass of MLCTs.

The oils and fats according to the present invention containing MCTs and MLCTs may include, for example, oils and fats (A) obtained by transesterifying a mixed oil which contains 65 to 95% by mass (preferably 78 to 92% by mass) of medium chain fatty acid triglycerides (MCTs) and 5 to 35% by mass (preferably 8 to 22% by mass) of long chain fatty acid triglycerides (LCTs), in which the content of n-decanoic acid contained in the constituent fatty acids of the medium chain fatty acid triglycerides is 60 to 75% by mass.

Also, the oils and fats according to the present invention containing MCTs and MLCTs is oils and fats (B) which contains 65 to 95% by mass (preferably 78 to 92% by mass) of medium chain fatty acid triglycerides (MCTs), and 5 to 35% by mass (preferably 8 to 24% by mass) of transesterified oil of MCTs and long chain fatty acid triglycerides (LCTs), in which the content of n-decanoic acid contained in the constituent fatty acids of the medium chain fatty acid triglycerides is 60 to 75% by mass.

Regardless of the above-described combinations, the preparation method of the present invention is not limited, as long as the content of tridecanoin is 30% by mass or less, the content of medium chain fatty acids contained in the constituent fatty acids is 65 to 95% by mass, and furthermore, the content of n-decanoic acid contained in the medium chain fatty acids is 60 to 75% by mass.

In an embodiment of the preparation of the oils and fats according to the present invention, MCTs, in which the mass ratio of caprylic acid:caproic acid contained in the constituent fatty acids is 4:6 to 2:8, are synthesized in an ordinary method. An example of the embodiment may include mixing 65 to 85 parts by mass of MCTs and 15 to 35 parts by mass of oils and fats (for example, rapeseed oil or sunflower oil) in which the content of unsaturated fatty acids contained in the constituent fatty acids is 70% by mass or more.

In another embodiment of the preparation of the oils and fats according to the present invention, MCTs, in which the mass ratio of caprylic acid:caproic acid contained in the constituent fatty acids is 4:6 to 2:8, are synthesized in an ordinary method. An example of the embodiment may include transesterifying 65 to 95 parts by mass of MCTs and 5 to 35 parts by mass of oils and fats (for example, rapeseed oil or sunflower oil) in which the content of unsaturated fatty acids contained in the constituent fatty acids is 70% by mass or more.

Further another embodiment of the preparation of the oils and fats according to the present invention is synthesizing, by an ordinary method, MCTs in which the mass ratio of caprylic acid:caproic acid contained in the constituent fatty acids is 4:6 to 2:8. For example, the following embodiment is included. In this embodiment, transesterification by an ordinary method is separately applied to a mixed oil of 65 to 95 parts by mass of MCTs and 5 to 35 parts by mass of fats and oils (for example, rapeseed oil or sunflower oil) in which the content of unsaturated fatty acids contained in the constituent fatty acids is 70% by mass or more. Then, 65 to 95 parts by mass of MCTs and 5 to 35 parts by mass of transesterified oil are mixed.

The oils and fats according to the present invention are clear for 24 hours at 5°C, and more preferably clear for 120 hours at 5°C. Being clear at 5°C refers to the state in which clouding and deposition of crystals are not identified when oils and fats containing oil and fat crystals which have been completely melted by heating at approximately 80°C are sampled in an amount of 50 g into a sample bottle, cooled to 20°C, and thereafter placed into a thermostatic chamber at 5°C ±0.5°C for time-dependent observation.

In the oils and fats according to the present invention, the content of the medium chain fatty acids contained in the constituent fatty acids is high, and the content of n-decanoic acid contained in the medium chain fatty acids is high. Therefore, even when high contents of the oils and fats according to the present invention are used in pharmaceutical agents, foods, and the like, which are intended for the physiological effect of medium chain fatty acids, there is little discomfort such as irritation of the pharynx or the upper stomach and heavy feelings in the stomach, when ingested.

The oils and fats according to the present invention are also oils and fats having excellent cold resistance. Therefore, particularly when used in an emulsion (food), even an emulsion (food) required to be kept refrigerated, always or only after opening, is unlikely to cause demulsification, thereby enabling excellent quality to be maintained. Examples of the emulsion (food) may include a liquid whitener for drinking, a filling, mayonnaise, a dressing, and a source.

### EXAMPLES

Next, the present invention will be described in further detail with reference to examples. However, the present invention is not limited to these examples.

### <Analysis Method>

The content of triglycerides was measured in accordance with AOCS Ce5-86.

The content of fatty acids was measured in accordance with AOCS Celf-96.

### <Raw Material Oils and Fats>

MCT 1: trade name: MCT C10R (constituent fatty acids: n-octanoic acid and n-decanoic acid, mass ratio thereof: 3:7), manufactured by The Nisshin OilliO Group, Ltd.

MCT 2: trade name: MCT ODO (constituent fatty acids: n-octanoic acid and n-decanoic acid, mass ratio thereof: 75:25), manufactured by The Nisshin OilliO Group, Ltd.

High oleic acid sunflower oil: trade name: Olein Rich, manufactured by Showa Sangyo Co., Ltd.

Transesterified oil 1: transesterified oil (MLCT content: 46% by mass) obtained by the reaction of a mixed oil of 14 parts by mass of MCT 2 and 86 parts by mass of rapeseed oil (trade name: Canola Oil, manufactured by The Nisshin OilliO Group, Ltd.) with sodium methylate as a catalyst

Transesterified oil 2: transesterified oil (MCT content: 80% by mass, MLCT content: 20% by mass) obtained by the reaction of a mixed oil of 90 parts by mass of MCT 1 and 10 parts by mass of high oleic acid sunflower oil with sodium methylate as a catalyst

Transesterified oil 3: transesterified oil (MCT content: 62% by mass, MLCT content: 37% by mass) obtained by the reaction of a mixed oil of 80 parts by mass of MCT 1 and 20 parts by mass of high oleic acid sunflower oil with sodium methylate as a catalyst

### <Preparation of Oils and Fats, and Cold Resistance Test>

According to the formulations described in Table 1 and Table 2, fats and oils of Reference Example 1, Comparative Example 1, and Examples 1 to 8 were prepared. The analysis result of fatty acids and triglycerides of the fats and oils was summarized in Table 1 and Table 2.

Also, the oils and fats prepared in each of Reference Example 1, Comparative Example 1, and Examples 1 to 8 were sampled in an amount of 50 g into a sample bottle, and heated at 80°C. Thereafter, the oils and fats were cooled to 20°C, and placed into a thermostatic chamber at 5°C ±0.5°C. The state was observed after 24 hours and after 120 hours. The observation result was summarized in Table 1 and Table 2.

**[Table 1]**

| Table 1 Preparation of oils and fats, and result of cold resistance test (values in % by mass) | | | | | |
|---|---|---|---|---|---|
| | Reference Example 1 | Comparative Example 1 | Example 1 | Example 2 | Example 3 |
| MCT 1 | - | 100 | 90 | 80 | 70 |
| MCT 2 | 100 | - | - | - | - |
| Sunflower oil | - | - | 10 | 20 | 30 |
| Transesterified oils and fats 1 | - | - | - | - | - |
| | - | - | - | - | - |
| Transesterified oils and fats 2 | - | - | - | - | - |
| | | | | | |
| Transesterified oils and fats 3 | | | | | |
| Total | 100 | 100 | 100 | 100 | 100 |
| Content of medium chain fatty acids in constituent fatty acids | 100 | 100 | 90 | 80 | 70 |
| Content of n-decanoic acid in medium chain fatty acids | 25 | 70 | 70 | 70 | 70 |
| Content of unsaturated fatty acids in constituent fatty acids | 0 | 0 | 9.2 | 18.4 | 27.6 |
| Content of tridecanoin | 1.1 | 30.9 | 27.1 | 24.1 | 21.1 |
| Content of MCTs | 100 | 100 | 90 | 80 | 70 |
| Content of MLCs | 0 | 0 | 0 | 0 | 0 |
| Evaluation result *1 | | | | | |
| 5°C after 24 hours | Excellent | Poor | Good | Excellent | Excellent |
| 5°C after 120 hours | Excellent | Poor | Poor | Poor | Excellent |

| | | | | | |
|---|---|---|---|---|---|
| *1 Excellent: clear Good: slightly clouding Fair: few crystals Poor: solidified | | | | | |

**[Table 2]**

| Table 2 Preparation of oils and fats, and result of cold resistance test | | | | | |
|---|---|---|---|---|---|
| (values in % by mass) | | | | | |

| | Example 4 | Example 5 | Example 6 | Example 7 | Example 8 |
|---|---|---|---|---|---|
| MCT 1 | 90 | 80 | 70 | - | - |
| MCT 2 | - | - | - | - | - |
| Sunflower oil | - | - | - | - | - |
| Transesterified oils and fats 1 | 10 | 20 | 30 | - | - |
| | - | - | - | 100 | - |
| Transesterified oils and fats 2 | - | - | - | - | 100 |
| | | | | | |
| Transesterified oils and fats 3 | | | | | |
| Total | 100 | 100 | 100 | 100 | 100 |
| Content of medium chain fatty acids in constituent fatty acids | 91.4 | 82.8 | 74.2 | 90 | 80 |
| Content of n-decanoic acid in medium chain fatty acids | 69.3 | 68.5 | 67.5 | 70 | 70 |
| Content of unsaturated fatty acids in constituent fatty acids | 7.9 | 15.8 | 23.7 | 9.2 | 18.4 |
| Content of tridecanoin | 27.1 | 24.1 | 21.1 | 24.1 | 18.7 |
| Content of MCTs | 90 | 80 | 70 | 80 | 62 |
| Content of MLCs | 4.6 | 9.2 | 13.8 | 20 | 37 |
| Evaluation result *1 | | | | | |
| 5°C after 24 hours | Excellent | Excellent | Excellent | Excellent | Excellent |
| 5°C after 120 hours | Excellent | Excellent | Excellent | Excellent | Excellent |

| | | | | | |
|---|---|---|---|---|---|
| *1 Excellent: clear Good: slightly clouding Fair: few crystals Poor: solidified | | | | | |

### <Manufacture of fillings>

According to the formulation of Table 3, with the oils and fats of Reference Example 1, Comparative Example 1, and Example 4, paste-form oil-in-water type emulsions (fillings) of Reference Example 2, Comparative Example 2, and Example 9 were respectively manufactured by a method known in the art. That is, a sugar alcohol solution and powder such as starch and protein are poured into a homomixer, and dispersed and dissolved. Thereafter, oils and fats heated to 85°C were poured, and stirred for emulsification. The emulsion was heated to 80°C, and retained under reduced pressure (0.048 MPa) while sometimes stirred. Cooling was further performed under reduced pressure (0.048 MPa).

**[Table 3]**

| Table 3. Formulation of filling | |
|---|---|
| (unit: % by mass) | |
| Oils and fats | 45.80 |
| Sugar alcohol solution | 44.65 |
| Starch | 3.90 |
| Egg white powder | 0.50 |
| Sweetened condensed milk | 3.20 |
| Skim milk | 0.50 |
| Processed egg yolk product | 0.67 |
| Milk flavor taste agent | 0.50 |
| Thickening polysaccharides | 0.10 |
| Flavors | 0.16 |
| Colorings | 0.02 |
| Total | 100.0 |

### <Evaluation of filling>

The melt-in-mouth of each filling and the feel of burden on the stomach caused by each filling of Reference Example 2, Comparative Example 2, and Example 9 were comprehensively evaluated by four evaluators who are sensitive to stimuli from medium chain fatty acids. The evaluation result was summarized in Table 4.

Also, the filling of each of Reference Example 2, Comparative Example 2, and Example 9 was refrigerated in a refrigerator at 5°C for two weeks. Thereafter, the state of the filling was observed. The observation result was summarized in Table 4.

### (Evaluation criteria of filling)

### Melt-in-mouth

| | |
|---|---|
| Moist and good melt-in-mouth | Excellent |
| Good melt-in-mouth | Good |
| Somewhat heavy | Fair |
| Oily and heavy | Poor |

### Feel of burden on stomach

| | |
|---|---|
| No feel of burden on stomach, and favorable | Excellent |
| Some feel of burden on stomach, but no awkwardness | Good |
| Considerable feel of burden on stomach | Poor |

### Observation of state

| | |
|---|---|
| Very favorable emulsification state maintained | Excellent |
| Favorable emulsification state maintained | Good |
| Demulsified | Poor |

**[Table 4]**

| Table 4. Evaluation result of filling | | | |
|---|---|---|---|
| | Reference Example 2 | Comparative Example 2 | Example 9 |
| Oils and fats used | Reference Example 1 | Comparative Example 1 | Example 4 |
| Evaluation result | | | |
| Melt-in-mouth | Good | Excellent | Excellent |
| Feel of burden on stomach | Poor | Good | Good |
| State after refrigeration | Excellent | Poor | Excellent |

Furthermore, the oils and fats according to the present invention may be the following first to sixth oils and fats.

The first oils and fats are oils and fats in which the content of tridecanoin is 30% by mass or less, the content of medium chain fatty acids in constituent fatty acids of the oils and fats is 65 to 95% by mass, and the content of n-decanoic acid in the medium chain fatty acids is 50% by mass or more.

The second oils and fats are the first oils and fats, in which the content of unsaturated fatty acids in the constituent fatty acids of the oils and fats is 3 to 35% by mass.

The third oils and fats are the first or second oils and fats which contain 30 to 93% by mass of medium chain fatty acid triglycerides (MCTs) and 3 to 70% by mass of medium and long chain fatty acid triglycerides (MLCTs).

The fourth oils and fats are any one of the first to third oils and fats which contain oils and fats obtained by transesterifying a mixed oil containing: 65 to 95% by mass of medium chain fatty acid triglycerides (MCTs) in which the content of n-decanoic acid in the constituent fatty acids is 50% by mass or more; and 5 to 35% by mass of long chain fatty acid triglycerides (LCTs).

The fifth oils and fats are any one of the first to third oils and fats which contain 65 to 95% by mass of medium chain fatty acid triglycerides (MCTs) in which the content of n-decanoic acid in the constituent fatty acids is 50% by mass or more, and 5 to 35% by mass of transesterified oil of MCTs and long chain fatty acid triglycerides (LCTs).

The sixth oils and fats are the first or second oils and fats which contain 65 to 95% by mass of medium chain fatty acid triglycerides (MCTs) and 5 to 35% by mass of long chain fatty acid triglycerides (LCTs).

Furthermore, the emulsion according to the present invention may be an emulsion which contains any one of the first to sixth oils and fats.

Furthermore, the food according to the present invention may be a food which contains any one of the first to sixth oils and fats.

Furthermore, the manufacturing method of oils and fats according to the present invention may be a manufacturing method of the first oils and fats, including a process of transesterifying a mixed oil containing: 65 to 95% by mass of medium chain fatty acid triglycerides (MCTs) in which the content of n-decanoic acid in the constituent fatty acids is 50% by mass or more; and 5 to 35% by mass of long chain fatty acid triglycerides (LCTs).

Furthermore, the manufacturing method of oils and fats according to the present invention may be a manufacturing method of the first oils and fats, including: a process of mixing 65 to 95% by mass of medium chain fatty acid triglycerides (MCTs) in which the content of n-decanoic acid in constituent fatty acids is 50% by mass or more; and 5 to 35% by mass of transesterified oil of MCTs and long chain fatty acid triglycerides (LCTs).

## Claims

1. Oils and fats, wherein the content of tridecanoin is 10-30% by mass, the content of medium chain fatty acids contained in constituent fatty acids is 65 to 95% by mass, and the content of n-decanoic acid contained in the medium chain fatty acids is 60 to 75% by mass.

2. The oils and fats according to claim 1, wherein the content of unsaturated fatty acids contained in the constituent fatty acids is 3 to 35% by mass.

3. The oils and fats according to claim 1 or 2, including 30 to 93% by mass of medium chain fatty acid triglycerides (MCTs), and 3 to 70% by mass of medium and long chain fatty acid triglycerides (MLCTs).

4. The oils and fats according to any one of claims 1 to 3, including oils and fats obtained by transesterifying a mixed oil which contains 65 to 95% by mass of medium chain fatty acid triglycerides (MCTs) and 5 to 35% by mass of long chain fatty acid triglycerides (LCTs) wherein the content of n-decanoic acid contained in constituent fatty acids of the medium chain fatty acid triglycerides is 60 to 75% by mass.

5. The oils and fats according to any one of claims 1 to 3, including 65 to 95% by mass of medium chain fatty acid triglycerides (MCTs) and 5 to 35% by mass of transesterified oil of MCTs and long chain fatty acid triglycerides, wherein the content of n-decanoic acid contained in constituent fatty acids of the medium chain fatty acid triglycerides is 60 to 75% by mass.

6. The oils and fats according to claim 1 or 2, including 65 to 95% by mass of medium chain fatty acid triglycerides (MCTs) and 5 to 35% by mass of long chain fatty acid triglycerides (LCTs).

## Patentansprüche

1. Öle und Fette, wobei der Gehalt an Tridecanoin 10-30 Masse-% beträgt, der in den Fettsäurebestandteilen enthaltene Gehalt an mittelkettigen Fettsäuren 65 bis 95 Masse-% beträgt und der in den mittelkettigen Fettsäuren enthaltene Gehalt an n-Decansäure 60 bis 75 Masse-% beträgt.

2. Öle und Fette nach Anspruch 1, wobei der in den Fettsäurebestandteilen enthaltene Gehalt an ungesättigten Fettsäuren 3 bis 35 Masse-% beträgt.

3. Öle und Fette nach Anspruch 1 oder 2, die 30 bis 93 Masse-% mittelkettige Fettsäuretriglyceride (MCTs) und 3 bis 70 Masse-% mittel- und langkettige Fettsäuretriglyceride (MLCTs) einschließen.

4. Öle und Fette nach einem der Ansprüche 1 bis 3, die Öle und Fette einschließen, welche durch Umesterung eines gemischten Öls erhalten werden, das 65 bis 95 Masse-% mittelkettige Fettsäuretriglyceride (MCTs) und 5 bis 35 Masse-% langkettige Fettsäuretriglyceride (LCTs) enthält, wobei der in den Fettsäurebestandteilen der mittelkettigen Fettsäuretriglyceride enthaltene Gehalt an n-Decansäure 60 bis 75 Massenprozent beträgt.

5. Öle und Fette nach einem der Ansprüche 1 bis 3, die 65 bis 95 Masse-% mittelkettige Fettsäuretriglyceride (MCTs) und 5 bis 35 Masse-% transesterifiziertes Öl aus MCTs und langkettigen Fettsäuretriglyceriden einschließen, wobei der in den Fettsäurebestandteilen der mittelkettigen Fettsäuretriglyceride enthaltene Gehalt an n-Decansäure 60 bis 75 Masse-% beträgt.

6. Öle und Fette nach Anspruch 1 oder 2, die 65 bis 95 Masse-% mittelkettige Fettsäuretriglyceride (MCTs) und 5 bis 35 Masse-% langkettige Fettsäuretriglyceride (LCTs) einschließen.

## Revendications

1. Huiles et graisses, dans lesquelles la teneur en tridécanoïne est de 10-30 % en masse, la teneur en acides gras à chaîne moyenne contenus dans des acides gras constitutifs est de 65 à 95 % en masse, et la teneur en acide n-décanoïque contenu dans les acides gras à chaîne moyenne est de 60 à 75 % en masse.

2. Huiles et graisses selon la revendication 1, dans lesquelles la teneur en acides gras insaturés contenus dans les acides gras constitutifs est de 3 à 35 % en masse.

3. Huiles et graisses selon la revendication 1 ou 2, comprenant de 30 à 93 % en masse de triglycérides d'acides gras à chaîne moyenne (MCT) et de 3 à 70 % en masse de triglycérides d'acides gras à chaîne moyenne et longue (MLCT).

4. Huiles et graisses selon l'une quelconque des revendications 1 à 3, comprenant des huiles et graisses obtenues par transestérification d'une huile mixte contenant de 65 à 95 % en masse de triglycérides d'acides gras à chaîne moyenne (MCT) et de 5 à 35 % en masse de triglycérides d'acides gras à chaîne longue (LCT), dans lesquelles la teneur en acide n-décanoïque contenu dans des acides gras constitutifs des triglycérides d'acides gras à chaîne moyenne est de 60 à 75 % en masse.

5. Huiles et graisses selon l'une quelconque des revendications 1 à 3, comprenant de 65 à 95 % en masse de triglycérides d'acides gras à chaîne moyenne (MCT) et de 5 à 35 % en masse d'huile transestérifiée de MCT et de triglycérides d'acides gras à chaîne longue, dans lesquelles la teneur en acide n-décanoïque contenu dans des acides gras constitutifs des triglycérides d'acides gras à chaîne moyenne est de 60 à 75 % en masse.

6. Huiles et graisses selon la revendication 1 ou 2, comprenant de 65 à 95 % en masse de triglycérides d'acides gras à chaîne moyenne (MCT) et de 5 à 35 % en masse de triglycérides d'acides gras à chaîne longue (LCT).
